# EUROPEAN PATENT SPECIFICATION

(11) **EP 3 513 739 B1**
(45) Date of publication and mention of the grant of the patent: **07.06.2023**
(21) Application number: 17851042.6
(22) Date of filing: 15.09.2017
(51) Int. Cl.: A61B 10/00, A61B 5/00

(54) **SYSTEM FOR DIAGNOSING CHRONIC PAIN-RELATED DISEASES AND DISEASES REQUIRING DIFFERENTIATION THEREFROM**
SYSTEM ZUR DIAGNOSE VON CHRONISCHEN SCHMERZASSOZIIERTEN KRANKHEITEN UND KRANKHEITEN, DIE EINE DIFFERENZIERUNG DAVON BENÖTIGEN
SYSTÈME DE DIAGNOSTIC DE MALADIES LIÉES À UNE DOULEUR CHRONIQUE ET MALADIES NÉCESSITANT UNE DIFFÉRENCIATION DE CES DERNIÈRES

(30) Priority: 16.09.2016 JP 2016181883
(43) Date of publication of application: 24.07.2019
(73) Proprietor: Osada, Kenichi, Kawasaki-shi, Kanagawa 215-0021 (JP)
(72) Inventor: Osada, Kenichi, Kawasaki-shi, Kanagawa 215-0021 (JP)
(74) Representative: Dr. Gassner & Partner mbB
(86) International application number: PCT/JP2017/033610
(87) International publication number: WO 2018/052145

(56) References cited:
- WO-A1-2013/125518
- LINDA C.J. OUDEJANS ET AL: "The influence of offset analgesia on the onset and offset of pain in patients with fibromyalgia :", PAIN., vol. 156, no. 12, 1 December 2015 (2015-12-01), pages 2521-2527, XP055678811, NL ISSN: 0304-3959, DOI: 10.1097/j.pain.0000000000000321
- MARIEKE NIESTERS ET AL: "Offset Analgesia in Neuropathic Pain Patients and Effectof Treatment with Morphine and Ketamine", ANESTHESIOLOGY., vol. 115, no. 5, 1 November 2011 (2011-11-01), pages 1063-1071, XP055771810, US ISSN: 0003-3022, DOI: 10.1097/ALN.0b013e31822fd03a
- BAR ET AL: "Decreased sensitivity to experimental pain in adjustment disorder", EUROPEAN JOURNAL OF PAIN, SAUNDERS, LONDON, GB, vol. 10, no. 5, 1 July 2006 (2006-07-01), pages 467-471, XP005454031, ISSN: 1090-3801, DOI: 10.1016/J.EJPAIN.2005.07.001

## Description

### [TECHNICAL FIELD]

The present invention relates to a system for noninvasively and conveniently diagnosing diseases requiring differentiation from chronic pain related diseases.

### [BACKGROUND ART]

Fibromyalgia is a disease characterized by the persistence of chronic pain over a wide range of the body; the prevalence rate is about 2% of the total population, and approximately 2 million people are considered to be affected in Japan. Regarding the current diagnostic criteria for fibromyalgia, those created by the American College of Rheumatology in 1990 are exclusively used. According to this, fibromyalgia is diagnosed when the pain over a wide range of the body continues for 3 months or more, and when the pain is felt at 11 points or more among 18 tender points on the whole body pressed with a force of 4 kg/cm².

In case of fibromyalgia in particular, there is no clear diagnostic criterion other than those mentioned above, and no other abnormal findings can be found in blood tests, *etc*., and therefore coupled with the low degree of recognition of the disease itself, it is said that fibromyalgia is a representative disease causing "doctor shopping" because subjects visit around 6 to 8 hospitals on average until they are diagnosed to have fibromyalgia.

Fibromyalgia is accompanied by various mental pains such as insomnia and depressed state, in addition to various chronic pain mainly of musculoskeletal muscle pain. Pain begins mainly in the body trunk and shoulder joints, gradually spreads widely to the muscles and connective tissues such as joints of the whole body, and correspondingly the degree of pain also intensifies. Along with the progress of the pain, not only deterioration of QOL but also daily living dysfunction is caused.

Functional somatic syndrome (FSS) which became advocated recently is characterized as follows: symptoms cannot be explained by obvious organic causes, and there are physical complaints, which are felt as pain, causing obstruction of daily life. Fibromyalgia is a disease included in this functional somatic syndrome. The pain of fibromyalgia is extremely severe compared to the pain of depression and psychogenic pain, *etc.,* daily life is gradually obstructed in many cases, and subjects may have a sleep disorder or become impossible to sit down due to the pain.

In addition, a disease image wherein the relationship between "pain" and "depressive state" deteriorates spirally is called "painful-depression". In other words, it is a state of so-called "a vicious cycle of pain and depressive state" in which the pain causes reduction of motivation, causing a depressive state, and it causes even stronger pain. In fibromyalgia, this "depressive state" is considered to be strongly involved in the disease state.

As described, "depression" and "fibromyalgia" are closely related to each other, and symptoms and therapeutic methods are similar; therefore it is difficult to differentiate them. With respect to fibromyalgia in particular, there is no diagnostic criterion other than the above-mentioned diagnostic criteria or no diagnostic indicator for which consensus has been obtained, and therefore diagnosis of fibromyalgia itself is currently difficult in the first place.

Recently, attempts to evaluate neurological disorders have been made by giving pain sensation stimulus by temperature stimulus or vibration stimulus. For example, in Non-Patent Documents 1 and 2, it is described that temperature stimulus was given to patients with neuropathic pain and patients with fibromyalgia, respectively, and that how the pain offset occurred in these patients was observed.

### [PRIOR ART REFERENCES]

### [NON-PATENT REFERENCES]

[NON-PATENT REFERENCE 1]
   Niesters et al., Anesthesiology. 2011 Nov; 115 (5): 1063-71.
[NON-PATENT REFERENCE 2]
   Oudejans et al., Pain. 2015 Dec; 156 (12): 2521-7.

### [SUMMARY OF INVENTION]

### [PROBLEMS TO BE SOLVED BY INVENTION]

An object of the present invention to provide a system for noninvasively and conveniently diagnosing diseases associated with chronic pain which are difficult to diagnose.

### [MEANS OF SOLVING PROBLEMS]

The inventor of the present invention has studied diseases causing chronic pain and the mechanism thereof, and has focused on fibromyalgia for which patients complain severe systemic pain despite that no neurological disorder is observed, and which significantly lowers the patients' QOL, and the diagnose of which is difficult, and the present inventor has conducted research on its mechanism and found that, in subjects suffering from fibromyalgia, an abnormality occurs in the transmission of pain sensation stimulus, in particular, in the offset of pain sensation stimulus. As the present inventor has continued to pursue more intensive studies, he has found that in subjects with chronic pain-related diseases, responses to pain sensation stimulus including offset of pain sensation stimulus differ in populations suffering from each disease including healthy individuals, and that affected diseases can be differentiated based on such differences in the responses; and the inventor has completed the present invention.

Namely, the present invention relates to the claims.

### [ADVANTAGEOUS EFFECTS OF INVENTION]

A simple test makes it possible to differentiate diseases related to chronic pain in a short time and noninvasively. In particular, it has become possible to differentiate fibromyalgia and chronic fatigue syndrome/myalgic encephalomyelitis which have been difficult to diagnose due to the lack of an indicator to be a specific biomarker for diagnosis, and possible to find obvious differences among fibromyalgia, chronic fatigue syndrome/myalgic encephalomyelitis, depression and rheumatism as well as healthy individuals, and accordingly, it has become possible to deepen the understanding of diseases such as fibromyalgia and chronic fatigue syndrome/myalgic encephalomyelitis and to give an appropriate diagnosis to patients suffering from chronic pain, including potential patients who have not yet been given definite diagnosis, and to perform optimal treatment.

### [BRIEF DESCRIPTION OF DRAWINGS]

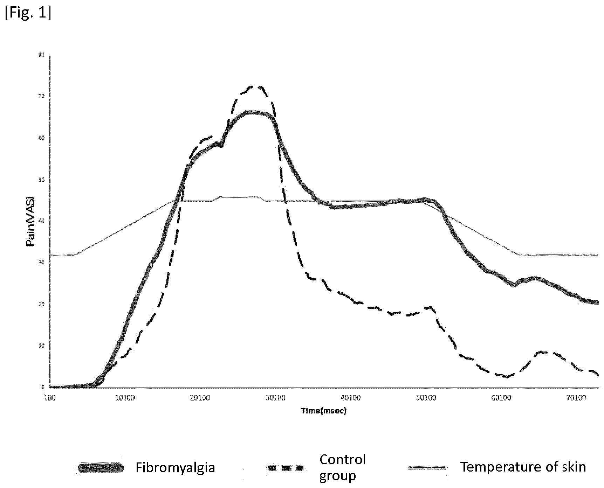

[Fig. 1] Figure 1 is a graph showing changes in the intensity of pain during the test in the healthy control group and the subject group suffering from fibromyalgia. It can be seen that pain offset occurred when the temperature is lowered from 46°C to 45°C, and the intensity of the pain felt remarkably decreases in the healthy control group, whereas in the subject group of fibromyalgia, the degree of offset is low and the degree of pain increases again with time after offset as compared with the healthy control group.

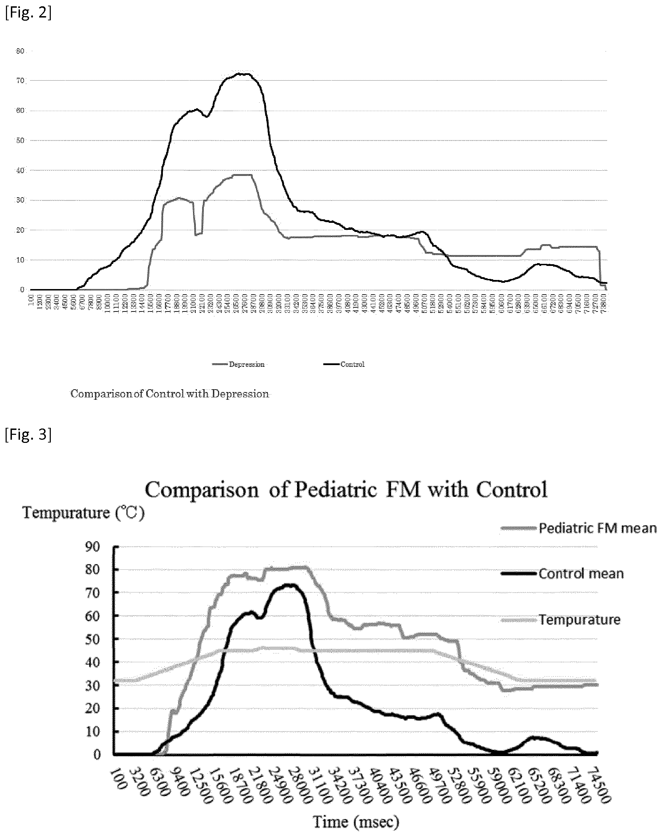

[Fig. 2] Figure 2 is a graph showing changes in the intensity of pain during the test in the healthy control group and the subject group suffering from depression. Although there is no big difference in the shape of the graphs, and the degrees of offsets are similar, it is clear that the degree of pain in the subject group of depression is apparently lower as a whole compared to the healthy control group.
[Fig. 3] Figure 3 is a graph showing changes in the intensity of pain during the test in the healthy control group and the subject group suffering from pediatric fibromyalgia. As with adult fibromyalgia, it can be seen that the degree of offset is also low in pediatric fibromyalgia.

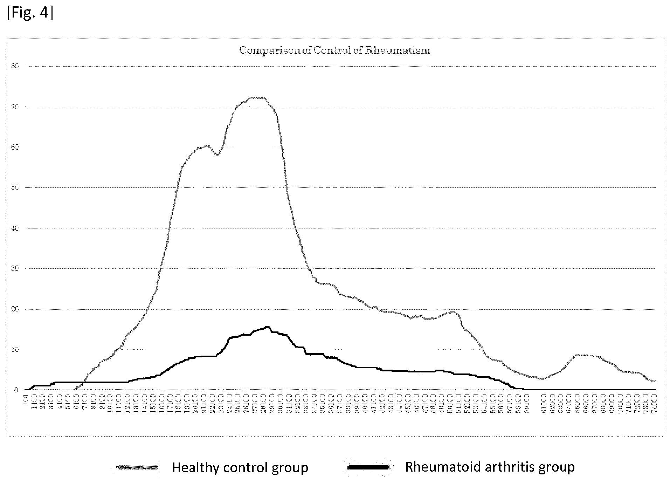

[Fig. 4] Figure 4 is a graph showing changes in the intensity of pain during the test in the healthy control group and the subject group suffering from rheumatoid arthritis. In the subject group of rheumatoid arthritis, it can be seen that the degree of pain is remarkably lower than the healthy control group in any section during the test.

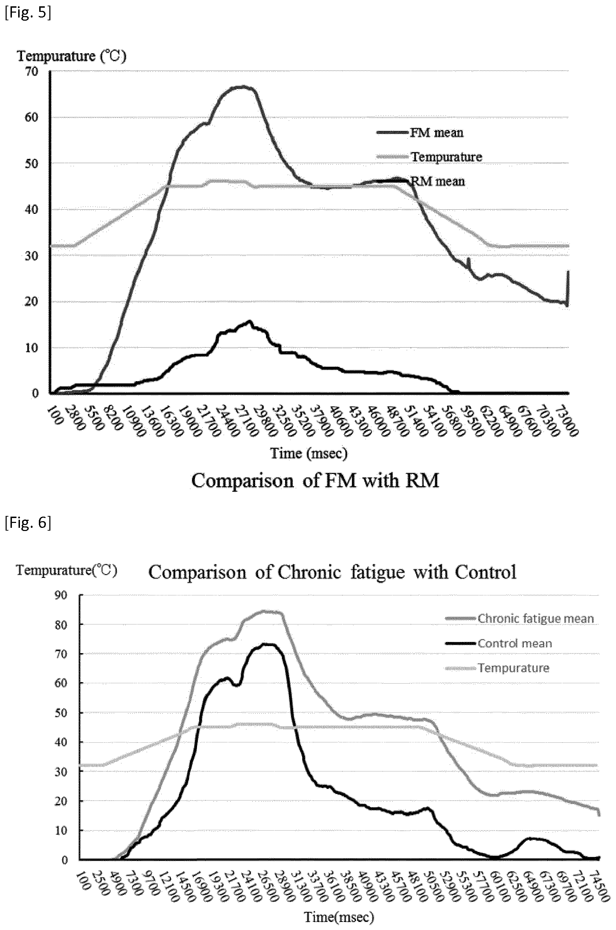

[Fig. 5] Figure 5 is a graph showing changes in the intensity of pain during the test in the subject group suffering from fibromyalgia and the subject group suffering from rheumatoid arthritis. The blue line (FM mean) represents the subject group of fibromyalgia and the black line (RM mean) represents the subject group of rheumatoid arthritis. As in the comparison with the healthy control group, it can be seen that the subject group of rheumatoid arthritis has a remarkably lower degree of pain compared with the subject group of fibromyalgia.
[Fig. 6] Figure 6 is a graph showing changes in the intensity of pain during the test in the healthy control group and the subject group suffering from chronic fatigue syndrome/myalgic encephalomyelitis. The orange line (Chronic fatigue mean) represents the subject group of chronic fatigue syndrome/myalgic encephalomyelitis, and the black line (Control mean) represents the healthy control group. Compared with the healthy control group, it can be seen that the subject group of chronic fatigue syndrome/myalgic encephalomyelitis shows higher degree of pain as a whole.

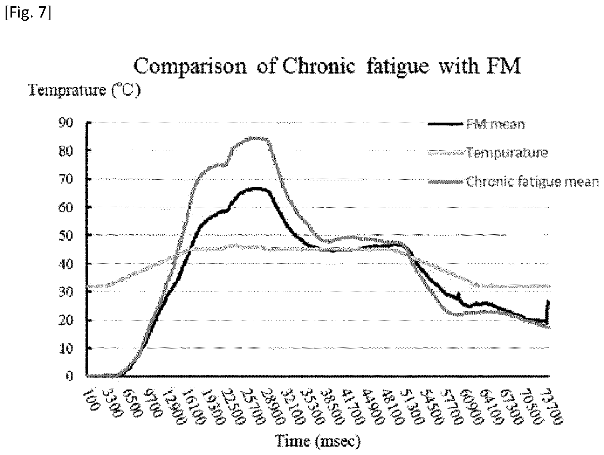

[Fig. 7] Figure 7 is a graph showing changes in the intensity of pain during the test in the subject group suffering from fibromyalgia and the subject group suffering from chronic fatigue syndrome/myalgic encephalomyelitis. The blue line (Chronic fatigue mean) represents the subject group of chronic fatigue syndrome/myalgic encephalomyelitis, and the black line (FM mean) represents the subject group of fibromyalgia. Compared with the subject group of fibromyalgia, it can be seen that the subject group of chronic fatigue syndrome/myalgic encephalomyelitis shows a significantly higher peak intensity of pain.

### [EMBODIMENTS FOR CARRYING OUT INVENTION]

Hereinafter, the present invention will be described in detail.

In the present invention, "chronic pain" is generally defined as "pain that persists beyond the time frame expected to be required for treatment, or pain associated with progressive non-cancerous diseases", and is classified into (1) nociceptive pain, (2) neuropathic pain, (3) complex chronic pain in which nociceptive pain and neuropathic pain coexist, (4) spontaneous chronic pain, and (5) psychogenic pain. It can also be classified broadly into, depending on its pathological mechanism, neuropathic pain, pain due to functional diseases and other pain.

In the present invention, the term "disease related to chronic pain" or "chronic pain-related disease" refers to a disease wherein one of its main symptoms is occurrence of chronic pain, either systemically or locally. Examples of such disease include, but are not limited to, fibromyalgia, peripheral neuropathic pain such as post herpetic neuralgia, diabetic neuropathy and phantom limb pain, central neuropathic pain such as pain after stroke and the like. A "disease requiring differentiation from chronic pain-related diseases" means a disease exhibiting a symptom similar to the above chronic pain diseases, but is not classified as a chronic pain-related disease, and its treatment is different from that of chronic pain diseases. Diseases requiring differentiation from chronic pain-related diseases include, but are not limited to, depression, chronic fatigue syndrome/myalgic encephalomyelitis, schizophrenia, somatic symptom disorder, degenerative arthritis, collagen diseases such as rheumatoid arthritis. The diagnostic system of the present invention may be applied to a subject having no particular neurological disorder, and such a subject includes a subject suspected of suffering from the following diseases: a disease requiring differentiation from chronic pain-related diseases namely depression, chronic fatigue syndrome/myalgic encephalomyelitis or rheumatism. Depression, rheumatism and chronic fatigue syndrome/myalgic encephalomyelitis are diseases requiring different therapeutic methods, but are difficult to differentiate.

In the present invention, "pain offset" is synonymous with "offset analgesia", meaning a phenomenon in which a slight reduction in noxious stimulus remarkably reduces the pain actually felt. Such phenomenon is known in the art (for example, see Hermans et al., Pain Physician 2016; 19:307 - 326. Therefore, "pain offset measurement test" means a test that qualitatively or quantitatively measures this offset phenomenon. Typically, pain assessment methods known in the art such as VAS and NRS are used to assess the pain experienced by a subject when the noxious stimulus is changed. The method of "pain offset measurement test" is known in the art (for example, see Niesters et al., Anesthesiology. 2011 Nov; 115 (5): 1063-71), and includes, but is not limited to, a method using a small fiber neuropathy evaluation device, *etc.* Conditioned pain modulation (CPM) is another known mechanism for relieving pain sensation including pain. Similar to the pain offset, CPM is also a mechanism to reduce pain that is felt as compared with the magnitude of actual stimulus, but it is known that CPM and pain offset are different in the following points (for example, Nahman-Averbuch et al., Pain 2014 December; 155 (12): 2491-2501, *etc*.):
(1) Opioids such as ketamine and tapentadol as well as NMDA receptor agonists act on CPM and decrease pain; however, they do not affect pain offset and there is no change in pain.
(2) When the activity of the brain is observed with fMRI, while in the CPM, the activities of the thalamus, insula, and the second sensory cortex (S2) decrease, whereas in the pain offset, the activity of the first sensory field (S1) decreases, and activities of the anterior insula, the dorsolateral prefrontal cortex, the intraparietal sulcus, and the inferior parietal lobule are remarkably enhanced as compared with CPM. Also in the CPM, the activity of the brainstem continues to decline, whereas in the pain offset, it continues to be enhanced.

Therefore, CPM is not included in the "pain offset" of the present invention.
"Small fiber neuropathy" means a disease having a disorder of small fibers of the peripheral nerves. In small fiber neuropathy, Aδ fibers and C fibers related to the transmission of pain sensation are impaired, and therefore symptoms such as reduction of warm pain sensation as well as pain are seen. "Small fiber neuropathy evaluation device" means a device capable of evaluating the disorder by stimulating small fibers by temperature stimulus and vibration stimulus, *etc.* Such devices are known in the art and are commercially available.

### <1 > EXAMINATION METHOD AND DIAGNOSTIC METHOD RELEVANT FOR THE PRESENT INVENTION

In the examination method, the pain offset in a subject is measured, and a judgment indicator is provided depending on the difference in the response, wherein the judgment indicator is for diagnosing whether or not the subject has a chronic pain-related disease and/or a disease requiring differentiation from chronic pain-related diseases, and when the subject is determined to have a disease, it is for diagnosing what type of disease the patient has.

The subject of the examination method is typically a human, because a degree of pain must be expressed. The subject to be examined may be a subject presumed to be healthy or a subject presumed to be suffering from some disease; it is preferably a subject who is complaining of chronic pain. In addition, even in a subject complaining of unknown pain, the examination method can provide judgement criteria for its causes, and therefore the method is more preferably performed to a subject who does not have neurological disorder, typically a subject who does not have anatomically neuropathic pain. Usually, a test using a small fiber neuropathy evaluation device is performed to assess the degree of chronic pain in a subject having such pain; it has been a surprising finding that, when a pain offset measurement test was performed by said device, particularly in subjects not having anatomical neurological disorder, differences in their responses occurred depending on the presence or absence of an affected disease or on the type of the affected disease.

The diagnostic method comprises the following steps (a) and (b), and optionally (c),
(a) performing a pain offset measurement test on a subject,
(b) analyzing the results obtained in the test of (a),
(c) comparing the analysis results obtained in (b) with a reference value.

In addition to the above, the diagnostic method of the present invention optionally includes the following step (d):
(d) determining the presence/absence and/or the type of a chronic pain-related disease and/or a disease requiring differentiation from chronic pain-related diseases based on the result of (c).

The noxious stimulus given to a subject in the "pain offset measurement test" in step (a) is not particularly limited as long as it is a stimulus in which its intensity can be arbitrarily changed, and examples thereof include temperature stimulus and vibration stimulus. In terms of convenient control of the intensity of stimulus, it is preferably temperature stimulus.

As described above, the "pain offset measurement test" is a qualitative and/or quantitative measurement, and it is preferably a quantitative measurement from the viewpoint of large amount of information obtained. Methods for quantitative assessment of pain are well known in the art and include, but are not limited to, Visual Analogue Scale (VAS), Numerical Rating Scale (NRS), Verbal Rating Scale (VRS), Face Scale, *etc.*

A problem in the quantitative assessment of pain is that the scale in the quantitative assessment of pain depends on a subject. For example, in VAS and NRS, we rely on subjective quantitative assessment of pain in which maximal pain is "the highest pain subject has ever experienced", so the assessment criteria may vary depending on the subject. The present inventor has made improvements to these conventional quantitative test methods, thereby making quantitative assessment more objective. That is, a reference stimulus (for example, a constant temperature stimulus) is given before the test and is set as a reference (for example, 5 in 10-level assessment), and the subject is asked to quantitatively assess the pain felt during the test; thus we have succeeded in reducing variations in the assessment due to individual differences such as subjects' experience and sensitivity.

In step (b), the results obtained in the above tests are analyzed in terms of various items by any methods known in the art. Examples of evaluation items include, but are not limited to, a difference in the value of pain between the peak and in the offset state after the peak, the value of pain of the peak itself, the time from the predetermined point in time (for example, at the start of the test or at the start of offset) to the time until the pain is no longer felt, and the degree of pain that increases again after the pain disappeared after the offset, *etc.* The results of the test may be visualized by a graph, *etc.*

In step (c), the reference value to be compared with the results analyzed above typically includes the value of the same item in healthy subjects. Such a value of the item may be a result obtained by performing the same test on a subject known to be healthy at the same time as the test on the subject to be examined, or it may be a mean value or a statistical median value in a population of healthy subjects which has been previously measured under a uniform condition. Furthermore, using a graph in which the test results are visualized and a graph in which reference values are visualized, and by comparing the shapes of the both graphs, visual comparison may be carried out.

As the reference value, a value of the same item in a subject known to be suffering from a predetermined chronic pain-related disease and/or disease requiring differentiation from chronic pain-related diseases is able to be used. Such a value of the item may be a result obtained by performing the same test on a subject known to be suffering from a predetermined chronic pain-related disease and/or disease requiring differentiation from chronic pain-related diseases, at the same time as the test on the subject to be examined, or it may be a mean value or a statistical median value, *etc.* in a population of subjects known to be suffering from a predetermined chronic pain-related disease and/or disease requiring differentiation from chronic pain-related diseases, which has been previously measured under a uniform condition. Furthermore, using a graph in which the test results are visualized and a graph in which reference values are visualized, and by comparing the shapes of the both graphs, visual comparison may be carried out. By setting a subject known to be suffering from a predetermined chronic pain-related disease and/or disease requiring differentiation from chronic pain-related diseases as a reference value, the chronic pain-related disease and/or the disease requiring differentiation from chronic pain-related diseases from which the subject to be examined is suffering can be easily distinguished. For example, when the shape of the graph visualizing the examination results of the subject to be examined is similar to the graph of the results of the same examination of a subject suffering from fibromyalgia, it is possible to determine that the subject to be examined is suffering from fibromyalgia.

The present inventor has found for the first time that, among the subjects not having anatomical neurological disorder, there is a subject whose result of pain offset test differs from the result of healthy subjects, and that the type of chronic pain-related diseases and/or diseases requiring differentiation from chronic pain-related diseases possessed by such subject can be differentiated using the fact that which of the items differs and that how it differs as the judgement criteria. For example, the intensity of a peak and the time to reach the peak are about the same as those of healthy controls, but the degree of the subsequent offset is significantly lower than that of healthy controls (that is, the pain felt at the time of offset is remarkably strong) and/or the time period until the pain is not felt is significantly longer than the value of healthy controls, then such subject can be determined that he/she suffers from fibromyalgia. Also, the present inventor has found that in subjects with fibromyalgia, as compared to healthy controls or compared to the subjects who are suffering from a disease classified as other neuropathic pain, the degree of pain that increases again after the post-offset pain disappeared significantly increases. Therefore, when the degree of such pain that increases again is significantly greater than that of healthy controls, it can also be determined that the subject is suffering from fibromyalgia, thus enabling the differentiation from diseases classified as other neuropathic pain.

Furthermore, when the time period of feeling pain and the degree of pain itself after offset are similar to those of healthy controls, but the level of the peak value of pain felt is significantly lower than that of healthy controls, such subject can be determined to be suffering from depression.

In addition, when the time period of feeling pain is similar to that of healthy controls, but both the level of the peak value of pain felt and the degree of pain after offset are significantly lower than those of healthy controls, such subject can be determined to be suffering from rheumatism.

Furthermore, when the time period of feeling pain is similar to that of healthy controls, but both the level of the peak value of pain felt and the degree of pain after offset are significantly higher than those of healthy controls, such subject can be determined to be suffering from chronic fatigue syndrome/myalgic encephalomyelitis.

As described above, when the degree of pain after offset is significantly higher than healthy controls, that is, when a subject shows low degree of offset and feels a significantly strong pain at the time of offset, then such subject can be considered to be suffering from fibromyalgia and/or chronic fatigue syndrome/myalgic encephalomyelitis. Accordingly, in another embodiment, when the degree of pain after offset is significantly higher than healthy controls, such subject can be determined to be suffering from fibromyalgia and/or chronic fatigue syndrome/myalgic encephalomyelitis.

Fibromyalgia has only one diagnostic criterion at present, and there is no indicator for which consensus is obtained as a biomarker. Since the above criterion requires the presence of long-term chronic pain of 3 months, it will take at least 3 months or more to confirm definite diagnosis of fibromyalgia. However, according to the diagnostic method found by the inventor, diagnosis can be made in a short time in a convenient and non-invasive manner.

Chronic fatigue syndrome/myalgic encephalomyelitis is also a disease accompanying a pain as a symptom, and no biological marker which can subjectively diagnose chronic fatigue syndrome/myalgic encephalomyelitis has been found to date. However, the present inventor has found for the first time that by measuring and analyzing the pain offset, a characteristic result is exhibited as compared with healthy controls and with subjects suffering from other chronic pain-related diseases such as fibromyalgia. Therefore, according to the method found by the inventor, the chronic fatigue syndrome/myalgic encephalomyelitis can also be accurately diagnosed in a short time in a convenient and non-invasive manner. In addition, it is thought that 20 to 50% of chronic fatigue syndrome/myalgic encephalomyelitis is a complication with fibromyalgia. Thus, in one embodiment, a subject suffering from or suspected of suffering from chronic fatigue syndrome/myalgic encephalomyelitis is not complicated with fibromyalgia.

In the treatment of fibromyalgia, conventional analgesics such as nonsteroidal anti-inflammatory drugs and opioids are generally not effective, and amitriptyline (a tricyclic antidepressant) and duloxetine (serotonin reuptake inhibitor: SNRI), which are specific antidepressants effective for pain, are effective. In addition, pregabalin is known to be effective. At present since the differentiation between fibromyalgia and depression is very difficult, in clinical practice, it is frequently admitted that fibromyalgia is misdiagnosed as depression and the subject is treated with an antidepressant that is not effective for pain, and does not improve. However, according to the method found by the inventor, both diseases can be more easily differentiated by responses to a pain offset assessment test, and appropriate treatment can be applied to patients.

Furthermore, rheumatism and chronic fatigue syndrome/myalgic encephalomyelitis are mentioned as other diseases that exhibit symptoms very similar to fibromyalgia and are difficult to differentiate. Since these diseases also have different effective treatments, misdiagnosis often leads to prolonged symptoms. However, according to the method found by the inventor, all of the fibromyalgia, rheumatism, chronic fatigue syndrome/myalgic encephalomyelitis and the above-mentioned depression can be differentiated by one test. Therefore, appropriate diagnosis and treatment can be rapidly applied to a patient considered to be suffering from a chronic pain-related disease and/or a disease requiring differentiation from chronic pain-related diseases.

The pain offset test will be described in detail below with reference to specific embodiments as examples. In one embodiment of the method found by the inventor, temperature stimulus is used as a noxious stimulus for use in pain offset measurement test. In such embodiments, the pain offset measurement test comprises the following steps (a-1) to (a-4) and optionally (a-5):
(a-1) a step of changing the temperature of a stimulus generation part from room temperature to a first temperature,
(a-2) a step of keeping the temperature of the stimulus generation part at the first temperature for a while,
(a-3) a step of changing the temperature of the stimulus generation part to a second temperature, and then keeping it for a while,
(a-4) a step of changing the temperature of the stimulus generation part to the first temperature, and then keeping it for a while,
(a-5) a step of returning the temperature of the stimulus generation part to room temperature.

In step (a-1), the temperature of the stimulus generation part (typically the probe of a small fiber neuropathy evaluation device) is changed from room temperature to the first temperature. The first temperature may be higher or lower than room temperature. When it is higher than room temperature, the noxious stimulus generated is a warm sensation stimulus, and when it is low, it is a cold sensation stimulus. The first temperature is not particularly limited, as long as it does not cause damage to the human body but gives a stimulus, and it includes, but is not limited to the following: when the temperature is higher than room temperature, it is about 40 to 45°C, preferably about 43 to 45°C, and more preferably about 45°C; when it is lower than room temperature, it is about 5 to 15°C, preferably about 5 to 10°C, and more preferably about 5°C, *etc.* Subsequently, in step (a-2), it is kept at the first temperature for a while.

In step (a-3), the temperature of the stimulus generation part is changed from the first temperature to the second temperature. The second temperature is a temperature that generates a noxious stimulus stronger than the first temperature. That is, when the noxious stimulus is a warm sensation stimulus, the second temperature is higher than the first temperature, and in the case of cold sensation stimulus, the second temperature is lower than the first temperature. The difference between the first temperature and the second temperature may be any value as long as a difference in the intensities of noxious stimulus is sufficient to cause a pain offset in the next step (a-3); and it is preferably about 1 to 5°C, more preferably about 1 to 3°C, and even more preferably about 1°C.

In step (a-4), the temperature of the stimulus generation part is changed from the second temperature to the first temperature. In such a step, the noxious stimulus is slightly reduced, and a pain offset phenomenon is observed in healthy subjects.

In step (a-5), the temperature of the stimulus generation part is changed from the first temperature to room temperature. In such a step, the noxious stimulus gradually decreases, and the pain felt also gradually decreases with it.

The time required for each step is not particularly limited, and a person skilled in the art can set an appropriate time. When the time is too short, it will be impossible to detect the pain in each step, and when it is too long, it will be undesirable because the test will last for a long time. Typically, but not limited to, for example, it is about 10 seconds in step (a-1), about 5 seconds in step (a-2), about 5 seconds in step (a-3), about 20 seconds in step (a-4), and about 10 seconds in step (a-5).

### <2> DIAGNOSTIC SYSTEM OF THE PRESENT INVENTION

The present invention provides a diagnostic system for examining the presence or absence of being suffering from a disease requiring differentiation from chronic pain-related diseases, and the type thereof, in non-invasive manner in a short time.

The diagnostic system of the present invention comprises (1) a temperature stimulus generation part, (2) an input part for inputting information relating to the temperature stimulus generated in the temperature stimulus generation part of (1), and (3) an analysis part for analyzing the inputted information of (2).

The temperature stimulus generation part is a part that applies various temperature stimuli to a subject by bringing the part into close contact with the subject's skin and changing its temperature. Therefore, anything may be used as long as it has a surface that can be brought into contact with the subject's skin and the surface has a structure to change its temperature. The temperature stimulus is a warm sensation stimulus. The temperature stimulus generated at the temperature stimulus generation part is generated by a temperature change of this part, and such temperature change is managed by a temperature change control program. The subject recognizes such temperature stimulus as a pain stimulus. Therefore, the temperature change control program controls pain stimulus given to the subject by controlling the temperature change of the temperature stimulus generation part. The temperature stimulus generation part may optionally have a sensor or the like for measuring temperature of the skin surface.

The temperature change control program is a program for controlling the temperature change due to the passage of time of the temperature stimulus generation part. In one embodiment of the present invention, the temperature change control program is capable of managing the temperature change of the temperature stimulus generation part in a unit of seconds from the start to the end of pain offset test in the above-mentioned examination/diagnostic methods. Therefore, the temperature control program is a program for controlling so as to appropriately provide temperature stimuli in the above-described pain offset test.

The temperature control program will be described in more detail by giving examples below; however, the present invention is not limited to such exemplified embodiments. A person skilled in the art can appropriately select temperature and time so as to suit the purpose of the person skilled in the art.

In one embodiment of the above-described pain offset test, the temperature stimulus generation part gradually changes the temperature to the first temperature, for example 45°C, for example in about 10 seconds with the start of the test. Thereafter, it is kept at the first temperature for a predetermined time, for example 5 seconds. The temperature stimulus generation part is then warmed to a second temperature, for example 46°C, where it is kept at the second temperature for a further predetermined time, for example 5 seconds. The temperature of the temperature stimulus generation part is then again lowered to the first temperature, where it is kept for a further predetermined time, for example 20 seconds. Thereafter, for example over a period of 10 seconds, the temperature of the temperature stimulus generation part is lowered to room temperature.

Therefore, as an example of a preferable embodiment of the present invention, the temperature control program functions such that the temperature of the temperature stimulus generation part is changed as follows: simultaneously with the start of a test, said temperature is increased to 45°C at 10 seconds after, and then kept at 45°C for 5 seconds, then increased to 46°C, kept at 46°C for 5 seconds, then lowered to 45°C, kept at 45°C for 20 seconds, then lowered to room temperature over 10 seconds to finish the test.

The input part is a part for inputting information relating to the temperature stimulus generated in the temperature stimulus generation part. "Information relating to temperature stimulus" includes not only the degree of warm sensation stimulus felt by a subject, but also the degree of pain stimulus when a temperature stimulus is recognized as a pain stimulus, and the like. The information may be entered directly by the subject to be diagnosed, or by the one performing the diagnosis, or may be automatically inputted by another measuring device. Since the stimulus given to the subject from the temperature stimulus generation part is basically a sensory stimulus, it is preferable that the input is made directly by the subject to be diagnosed. The input device may be an ordinary input device used in the art, and it includes, but is not limited to, a keyboard, a barcode reader, a touch panel, *etc.,* as well as a button, a switch, a slider lever or the like.

The analysis part is a part for analyzing the information inputted from the input part and comparing it with the reference information. Based on the result of such comparison, it becomes possible to diagnose whether the subject to be diagnosed is suffering from a chronic pain-related disease and/or a disease requiring differentiation from chronic pain-related diseases, and when the subject is suffering, what type of disease it is. In one embodiment of the present invention, the analysis may be the same as the analysis in step (b) in the above diagnostic method. Therefore, examples of the analysis include, but are not limited to, the difference between the peak value of the pain and the value in the offset state after the peak, the value of the peak itself, and a time period from a predetermined time point (for example, at the start of the test or at the start of offset) until the pain is no longer felt. The results of the test may be visualized by a graph, *etc.* The analysis part is an arithmetic processing device known in the art, for example, a processor, a microprocessor, *etc.*

In addition to the above, the system of the present invention may further include other parts, for example, an output part for outputting analysis results and/or comparison results, a recording part for recording necessary data and programs, and a processing part that performs arithmetic processing for operating the system of the present invention, such as executing various programs that may include a temperature control program, a data analysis program, and a data comparison program, *etc.* In addition, the respective parts constituting the system of the present invention may be integrated as one part. For example, the output part and the input part, as well as the analysis part and the processing part may be the same parts.

The output part may be anything as long as analysis result and/or comparison result can be outputted to the outside, and it may be, for example, a display or a projector that outputs electronically, as well as a printer, *etc.* that outputs to paper, *etc.*

According to the system of the present invention, it is possible to noninvasively and conveniently diagnose whether a subject to be diagnosed is suffering from a disease requiring differentiation from chronic pain-related diseases, and when the subject is suffering, what type of disease it is. In particular, in some of the chronic pain-related diseases and diseases requiring differentiation therefrom, causes are hard to be clarified or unknown, and there are no definite biological markers; nonetheless, some diseases require therapeutic discrimination. According to the system of the present invention, each of these diseases can be accurately diagnosed and differentiated. As mentioned above, although depression, rheumatism and chronic fatigue syndrome/myalgic encephalomyelitis are diseases requiring therapeutic discrimination, it is difficult to differentiate them, and with respect to chronic fatigue syndrome/myalgic encephalomyelitis, there is currently no biological marker for which consensus has been obtained. However, in the system of the present invention, all of these diseases can be conveniently differentiated as well. Therefore, in the system of the present invention, preferably the subject is suffering from at least one selected from depression, rheumatism and chronic fatigue syndrome/myalgic encephalomyelitis.

Hereinafter, the present invention will be specifically described with reference to examples, but the present invention is not limited to these examples. All examples referring to fibromyalgia are comparative examples not related to the invention.

### [EXAMPLES]

In the following examples, "COVAS" means a value obtained in a pain offset measurement test by quantifying a degree of pain wherein the strongest pain experienced up to now is set as 100. In addition, in the following analysis, the period from 16000 msec to 30000 msec is a period from the time when the temperature is first raised to 45°C, further raised to 46°C, and then lowered to 45°C; the analysis of COVAS during this period mainly means analysis of "peak intensity of pain". Iso, the period from 30000 msec to 60000 msec is a period from the time when the temperature is lowered from 46°C to 45°C, maintained at 45°C for a while, and then started to be lowered to room temperature; the analysis of COVAS during this period mainly means analysis of "intensity of pain after offset".

### EXAMPLE 1. PAIN OFFSET MEASUREMENT AND ANALYSIS

(1) Subject to be tested:
   As subjects to be tested, 17 healthy controls, 133 subjects diagnosed as suffering from fibromyalgia based on the criteria created by the American College of Rheumatology in 1990, and 9 subjects suffering from depression who complain chronically strong pain as one of the symptoms were selected, and a pain offset measurement test was conducted for each subject.
(2) Pain offset measurement test:
   Prior to the start of the test, a temperature stimulus generation part (hereinafter referred to as probe) of a small fiber neuropathy evaluation device was put on the forearm part of the subject, the temperature was once raised to 46°C, and stimulus was applied for 5 seconds. After returning to room temperature and waiting for a while, the probe was again put on the forearm of the subject, and the temperature of the probe was linearly raised to 45°C. Stimulus was applied at 45°C for 5 seconds, the temperature was immediately raised to 46°C, stimulus was applied for another 5 seconds, then the temperature was returned to 45°C again and stimulus was applied for 20 seconds. Thereafter, the temperature of the probe was linearly lowered to room temperature. With the strongest pain experienced up to now as 100, the subject was asked to evaluate the degree of pain during the test using a value of 0 to 100.
(3) Analysis of results:
   Changes in the mean value of the degree of pain in each subject group at each time point during the test were plotted on a graph, and shown in Fig. 1 and Fig. 2.

Time points from 30000 msec to 60000 msec after the start of the test were sectioned every 10000 msec, and in each section comparisons were made by t-test between the pain intensity of the fibromyalgia subject group and the pain intensity of the healthy control group. As a result, the most significant difference (p<0.01) was observed in the section of 50000 to 60000 msec (Fig. 1). In addition, regarding the blood flow around the stimulus generation part, no change was observed in the healthy control group, whereas a decrease in blood flow was observed in the fibromyalgia subject group during pain.

In addition, comparison was made by t-test between the pain intensity of the fibromyalgia subject group and the pain intensity of the healthy control group, in each section of 30000 msec to 45000 msec, 45000 msec to 60000 msec, and 30000 msec to 60000 ms after the start of the test. The results are shown in Table 1.

**[Table 1]**

| Comparison of FM and healthy control. | | | | | | | |
|---|---|---|---|---|---|---|---|
| | | N | M | SD | t | r | P |
| 30000-45000 | FM | 133 | 48.57 | 34.39 | 5.81 | 0.71 | 0.00 |
| | Control | 17 | 17.56 | 18.25 | | | |
| 45000-60000 | FM | 133 | 38.84 | 33.04 | 9.08 | 0.70 | 0.00 |
| | Control | 17 | 5.85 | 9.22 | | | |
| 30000-60000 | FM | 133 | 43.78 | 32.61 | 7.87 | 0.73 | 0.00 |
| | Control | 17 | 11.71 | 12.09 | | | |

| | | | | | | | |
|---|---|---|---|---|---|---|---|
| (FM: Fibromyalgia group, C: Healthy control group) | | | | | | | |

As shown in Table 1, there were statistically significant increases in the pain after offset in the fibromyalgia group compared to the healthy control group in all the periods. Therefore, the pain after offset was higher in the fibromyalgia group than in the healthy control group, and the differentiation between the two groups was possible.

Similarly, in the healthy control group and the depression subject group, the pain intensity at peak time was compared. As a result, a significant decrease in the peak intensity (about 50 to 60% relative to the healthy control) was confirmed in the depression subject group (Fig. 2).

In addition, comparison was made by t-test between the pain intensity of the depression subject group and the pain intensity of the healthy control group, in each section of 16000 msec to 30000 msec, 30000 msec to 45000 msec, 45000 msec to 60000 msec, and 30000 msec to 60000 ms after the start of the test. The results are shown in Table 2.

**[Table 2]**

| Comparison of depression and healthy control. | | | | | | | |
|---|---|---|---|---|---|---|---|
| | | N | M | SD | t | df | p |
| 16000-30000 | C | 17 | 60.79 | 28.61 | 2.32 | 24 | .029 |
| | D | 9 | 30.80 | 36.35 | | | |
| 30000-45000 | C | 17 | 27.53 | 22.60 | 0.83 | 24 | .415 |
| | C | 9 | 18.60 | 32.13 | | | |
| 45000-60000 | C | 17 | 12.34 | 16.01 | -0.18 | 24 | .859 |
| | D | 9 | 13.82 | 25.96 | | | |
| 30000-60000 | C | 17 | 19.94 | 18.09 | 0.41 | 24 | .688 |
| | D | 9 | 16.20 | 28.92 | | | |

| | | | | | | | |
|---|---|---|---|---|---|---|---|
| (D: Depression group, C: Healthy control group) | | | | | | | |

As shown in Table 2, the COVAS value of the depression subject group was significantly lower than that of the healthy control group at 16000 msec to 30000 msec. Therefore, since the peak pain at the time of offset was lower than that of the healthy control group, differentiation between the two groups was possible.

Furthermore, comparison was made by t-test between the pain intensity of the fibromyalgia subject group and the pain intensity of the depression subject group, in each section of 30000 msec to 45000 msec, 45000 msec to 60000 msec, and 30000 msec to 60000 ms after the start of the test. The results are shown in Table 3.

**[Table 3]**

| Comparison of FM and depression. | | | | | | | |
|---|---|---|---|---|---|---|---|
| | | N | M | SD | t | r | P |
| 30000-45000 | FM | 133 | 48.57 | 34.39 | 2.54 | 0.21 | 0.01 |
| | D | 9 | 18.60 | 32.13 | | | |
| 45000-60000 | FM | 133 | 38.84 | 33.04 | 2.75 | 0.58 | 0.02 |
| | D | 9 | 13.82 | 25.96 | | | |
| 30000-60000 | FM | 133 | 43.78 | 32.61 | 2.75 | 0.67 | 0.02 |
| | D | 9 | 16.20 | 28.92 | | | |

| | | | | | | | |
|---|---|---|---|---|---|---|---|
| (FM: Fibromyalgia group, D: Depression group) | | | | | | | |

As shown in Table 3, in all the sections, the COVAS value was statistically significantly higher in the fibromyalgia group than in the depression group. Therefore, the pain after offset was higher in fibromyalgia than in depression, and differentiation between the two groups was possible.

### EXAMPLE 2. (NOT ACCORDING TO THE INVENTION) DETAILED ANALYSIS OF PAIN OFFSET IN SUBJECTS WITH FIBROMYALGIA

(1) Subject to be tested:
For 117 subjects who were evaluated as having fibromyalgia using the Japanese version of Fibromyalgia Questionnaire (J-FIQ) that is currently used as rating scale for fibromyalgia, a pain offset measurement test was conducted as in Example 1, and the results were analyzed.
(2) Correlation analysis between evaluation value of J-FIQ and value in section of 35000 to 40000 msec in pain offset test:
Correlation analysis was performed to see the correlation between the evaluation value of J-FIQ and the mean value of pain value after offset (COVAS) in the section of 35000 to 40000 msec, and a correlation weaker than r = 0.319 (p<0.001) was observed. Namely, it is suggested that, when the value of J-FIQ is high, the mean value of the pain value after offset (COVAS) in the section of 35000 to 40000 msec is also high.

**[Table 4]**

| Correlation between J-FIQ and COVAS value at 35000 to 45000 msec. | | | | | |
|---|---|---|---|---|---|
| | N | M | SD | p | r |
| J-FIQ | 117 | 63.02 | 25.54 | <0.001 | 0.319 |
| COVAS | 117 | 43.61 | 36.83 | | |

(3) Correlation analysis of evaluation value of J-FIQ and pain value after offset (COVAS) in section of 45000 to 60000 msec:
Correlation analysis was performed to see the correlation between the evaluation value of J-FIQ and the mean value of the pain value after offset (COVAS) in the section of 45000 to 60000 msec, and a correlation weaker than r = 0.343 (p<0.001) was observed. Namely, it is suggested that, when the value of J-FIQ is high, the mean value of the pain value after offset (COVAS) in the section of 45000 to 60000 msec is also high.

**[Table 5]**

| Correlation between J-FIQ and COVAS value at 45000 to 60000 msec. | | | | | |
|---|---|---|---|---|---|
| | N | M | SD | p | r |
| J-FIQ | 115 | 41.65 | 34.73 | <0.001 | 0.343 |
| COVAS | 115 | 62.82 | 25.70 | | |

(4) Correlation analysis for each item of evaluation value of J-FIQ and pain value after offset (COVAS):
The correlation between J-FIQ and each of pain values after offset (COVAS) at 30000 to 45000 msec, 45000 to 60000 msec, and 30000 to 60000 msec was analyzed in terms of each item. There was also a correlation with the total value, suggesting that the higher the pain value after offset (COVAS), the higher the values of J - FIQ. The correlation coefficients confirming the correlation between each item of J-FIQ and the pain value after offset (COVAS) are summarized in the table below. Meanwhile, the values of the State-Trait Anxiety Inventory (STAI) and the Beck Depression Inventory (BDI) were not correlated with the pain value after offset (COVAS).

**[Table 6]**

| Correlation between each item of J-FIQ and COVAS value at each section. | | | |
|---|---|---|---|
| Item | Correlation coefficient | | |
| | 30000-45000 msec | 45000-60000 msec | 30000-60000 msec |
| I can go shopping | 0.365 | 0.410 | 0.398 |
| I can wash using a washing machine | 0.368 | 0.350 | 0.368 |
| I can prepare meals | - | 0.300 | 0.279 |
| I can wash dishes and cookware by hands | 0.343 | 0.381 | 0.371 |
| I can use a vacuum cleaner | 0.302 | 0.374 | 0.347 |
| I can set up Japanese-style bedding and make up bed | - | 0.259 | - |
| I can walk several hundred meters | 0.371 | 0.402 | 0.397 |
| I can visit friends and relatives | 0.289 | 0.333 | 0.319 |
| I can work in the garden | 0.378 | 0.440 | 0.424 |
| I can drive a car | 0.441 | 0.420 | 0.442 |
| Number of days when I felt good | -0.353 | -0.446 | -0.410 |
| Number of days off work | 0.542 | 0.492 | 0.531 |
| Hindrance to daily life due to pain | 0.406 | 0.392 | 0.409 |
| Intensity of pain | 0.454 | 0.436 | 0.457 |
| Intensity of tiredness | 0.334 | 0.284 | 0.317 |
| Intensity of morning weariness | 0.401 | 0.405 | 0.414 |
| Intensity of stiffness | 0.368 | 0.357 | 0.372 |
| Intensity of anxiety | 0.338 | 0.265 | 0.310 |
| Intensity of depressed mood | 0.285 | 0.273 | 0.286 |
| J-FIQ total point | 0.454 | 0.435 | 0.456 |

From these results, it became clear that the values of pain after offset (COVAS) in all the sections correlate with the clinical symptoms of fibromyalgia. Until now, since no objective indicator of pain correlated with the clinical symptoms has been found in fibromyalgia, COVAS is the first indicator that has been found for the first time to be very useful as a biological marker of symptoms of fibromyalgia. Therefore, it is considered that fibromyalgia can be accurately diagnosed by analyzing COVAS value quantified by the measurement of pain offset.

### EXAMPLE 3. (NOT ACCORDING TO THE INVENTION) PAIN OFFSET MEASUREMENT TEST FOR PEDIATRIC FIBROMYALGIA

Pediatric fibromyalgia is known to be more difficult to diagnose compared to fibromyalgia of adults. Therefore, the biological marker found in Example 2 was tested in terms of whether it could also function in evaluating pediatric fibromyalgia.
(1) Subject to be tested:
   Five children who were diagnosed as having fibromyalgia in the pediatrics department of Yokohama City University Hospital and 17 subjects as healthy controls were selected, and a pain offset measurement test was conducted in the same way as in Example 1 and the results were analyzed.
(2) Result:
   The results are shown in Fig. 3. Compared to the healthy controls, the pain intensity was clearly greater in the fibromyalgia group as a whole.

Next, the mean values of COVAS of the healthy controls and the pediatric fibromyalgia group in the section of 30000 to 60000 msec were analyzed by t-test. The results are shown in the table below.

**[Table 7]**

| 30000-60000msec | | | | | |
|---|---|---|---|---|---|
| | N | M | SD | t | P |
| control | 17 | 19.94 | 18.09 | 2.95 | 0.008 |
| FM | 5 | 52.17 | 31.61 | | |

| | | | | | |
|---|---|---|---|---|---|
| (FM: Fibromyalgia group, control: Healthy control group) | | | | | |

From these results, in children, it was confirmed that the COVAS of the fibromyalgia group was significantly larger in the section of 30000 to 60000 msec as compared with the healthy control group. Therefore, COVAS was confirmed to be a useful biological marker also in pediatric fibromyalgia. Namely, it is considered that pediatric fibromyalgia can be diagnosed by analyzing results of a pain offset measurement test.

### EXAMPLE 5. PAIN OFFSET MEASUREMENT TEST ON RHEUMATOID ARTHRITIS

Evaluation test of offset pain was conducted on subjects with rheumatoid arthritis, which is a disease exhibiting very similar symptoms as fibromyalgia and requiring differentiation therefrom, and the results were compared with the evaluation test results of the fibromyalgia group.
(1) Subject to be tested:
   133 subjects in the fibromyalgia group, 10 subjects in the rheumatoid arthritis group, and 17 subjects in the healthy control group were selected, and the pain offset measurement test was conducted in the same manner as in Example 1, and the results were analyzed.
(2) Comparison with healthy control group:
   The results are shown in Fig. 4. Compared to the healthy control group, the pain intensity was clearly small as a whole in the rheumatoid arthritis group.

Next, the mean values of COVAS of the healthy control group and the rheumatoid arthritis group in each section of 16000 msec to 30000 msec, 30000 to 45000 msec, 45000 to 60000 msec, and 30000 to 60000 msec were analyzed by t-test. The results are shown in the table below.

**[Table 8]**

| Comparison of rheumatism and healthy control. | | | | | | | |
|---|---|---|---|---|---|---|---|
| | | N | M | SD | t | df | p |
| 16000-30000 | C | 17 | 60.79 | 28.61 | 5.98 | 24.85 | 0.00 |
| | R | 10 | 10.34 | 15.15 | | | |
| 30000-45000 | C | 17 | 27.53 | 22.60 | 2.47 | 25.00 | 0.02 |
| | R | 10 | 7.55 | 15.76 | | | |
| 45000-60000 | C | 17 | 12.34 | 16.01 | 2.10 | 23.39 | 0.05 |
| | R | 10 | 3.01 | 6.79 | | | |
| 30000-60000 | C | 17 | 19.94 | 18.09 | 2.30 | 25.00 | 0.03 |
| | R | 10 | 15.28 | 11.25 | | | |

| | | | | | | | |
|---|---|---|---|---|---|---|---|
| (R: Rheumatism group, C: Healthy control group) | | | | | | | |

It was confirmed that the COVAS value of the subjects of the rheumatoid arthritis group was significantly smaller than the COVAS value of the healthy control group in any of the sections 16000 to 30000 msec, 30000 to 45000 msec, 45000 to 60000 msec, and 30000 to 60000 msec. Therefore, rheumatoid arthritis can be easily diagnosed by the measurement of COVAS.

(3) Comparison with fibromyalgia group:
The results are shown in Fig. 5. Compared with the fibromyalgia group, the pain intensity was apparently small in the rheumatoid arthritis group as a whole.

Next, the mean values of COVAS of the fibromyalgia group and the rheumatoid arthritis group in each section of 30000 to 45000 msec, 45000 to 60000 msec, and 30000 to 60000 msec were analyzed by Welch's t-test. The results are shown in the table below.

**[Table 9]**

| Comparison of fibromyalgia and rheumatism. | | | | | | | |
|---|---|---|---|---|---|---|---|
| | | N | M | SD | t | r | P |
| 30000-45000 | FM | 133 | 48.57 | 34.39 | 7.06 | 0.87 | 0.00 |
| | RM | 10 | 7.55 | 15.76 | | | |
| 45000-60000 | FM | 133 | 38.84 | 33.04 | 10.01 | 0.80 | 0.00 |
| | RM | 10 | 3.01 | 6.79 | | | |
| 30000-60000 | FM | 133 | 43.78 | 32.61 | 8.47 | 0.87 | 0.00 |
| | RM | 10 | 5.28 | 11.24 | | | |

| | | | | | | | |
|---|---|---|---|---|---|---|---|
| (FM: Fibromyalgia group, RM: Rheumatism group) | | | | | | | |

It was confirmed that the COVAS value of the subjects of the rheumatoid arthritis group was significantly smaller than the COVAS value of the subjects of the fibromyalgia group in any of the sections 30000 to 45000 msec, 45000 to 60000 msec, and 30000 to 60000 msec. Therefore, fibromyalgia and rheumatoid arthritis can be easily differentiated by the measurement of COVAS.

### Example 6. PAIN OFFSET MEASUREMENT TEST ON CHRONIC FATIGUE SYNDROME/MYALGIC ENCEPHALOMYELITIS GROUP

Evaluation test of offset pain was conducted on subjects with chronic fatigue syndrome/myalgic encephalomyelitis which is a disease requiring differentiation from fibromyalgia and depression, and the results were compared with the evaluation test results of the healthy control group and fibromyalgia group.
(1) Subject to be tested:
   90 subjects who were diagnosed with chronic fatigue syndrome/myalgic encephalomyelitis at Osaka City University Hospital Fatigue Clinical Center were selected, and together with 133 subjects with fibromyalgia and 17 healthy controls, they were subjected to a pain offset measurement test in the same manner as in Example 1, and the results were analyzed.
(2) Comparison with healthy controls:
   The results are shown in Fig. 6. Compared to the healthy controls, the subjects of chronic fatigue syndrome/myalgic encephalomyelitis showed overall high pain intensity.

Next, the mean values of COVAS of the healthy controls and the subjects of chronic fatigue syndrome/myalgic encephalomyelitis in each section of 30000 to 45000 msec, 45000 to 60000 msec, and 30000 to 60000 msec were analyzed by Welch's t-test. The results are shown in the table below.

**[Table 10]**

| 30000-45000msec | | | | | |
|---|---|---|---|---|---|
| | N | M | SD | t | P |
| control | 17 | 27.53 | 22.5 | 4.32 | <.000 |
| control fatigue | 90 | 54.87 | 30.47 | | |

| | | | | | |
|---|---|---|---|---|---|
| (chronic fatigue: Chronic fatigue syndrome/myalgic encephalomyelitis group, control: Healthy control group) | | | | | |

**[Table 11]**

| 45000-60000msec | | | | | |
|---|---|---|---|---|---|
| | N | M | SD | t | P |
| control | 17 | 12.34 | 16.01 | 5.07 | <.000 |
| control fatigue | 90 | 38.09 | 31.09 | | |

| | | | | | |
|---|---|---|---|---|---|
| (chronic fatigue: Chronic fatigue syndrome/myalgic encephalomyelitis group, control: Healthy control group) | | | | | |

**[Table 12]**

| 30000-60000msec | | | | | |
|---|---|---|---|---|---|
| | N | M | SD | t | P |
| control | 17 | 19.94 | 18.09 | 4.92 | <.000 |
| control fatigue | 90 | 46.47 | 29.8 | | |

| | | | | | |
|---|---|---|---|---|---|
| (chronic fatigue: Chronic fatigue syndrome/myalgic encephalomyelitis group, control: Healthy control group) | | | | | |

It was confirmed that the COVAS value of the subjects of chronic fatigue syndrome/myalgic encephalomyelitis was significantly larger than the COVAS value of the healthy controls in any of the sections 30000 to 45000 msec, 45000 to 60000 msec, and 30000 to 60000 msec. Therefore, it was suggested that COVAS could be a useful biological marker also in chronic fatigue syndrome/myalgic encephalomyelitis.

Furthermore, a pain offset measurement test was conducted as in Example 1 for 50 subjects of healthy control group and 91 subjects of the chronic fatigue syndrome/myalgic encephalomyelitis group, and the mean values of COVAS of the healthy control group and the subjects of the chronic fatigue syndrome/myalgic encephalomyelitis group in the section of 16000 msec to 30000 msec were analyzed by t-test. The results are shown in the table below.

**[Table 13]**

| Comparison of chronic fatigue syndrome/myalgic encephalomyelitis and healthy control. | | | | | | | |
|---|---|---|---|---|---|---|---|
| | | N | M | SD | t | df | p |
| 16000-30000 | C | 50 | 42.82 | 30.97 | -5.632 | 81.424 | 0.00 |
| | CFS | 91 | 71.22 | 23.84 | | | |

| | | | | | | | |
|---|---|---|---|---|---|---|---|
| (CFS: Chronic fatigue syndrome/myalgic encephalomyelitis group, C: Healthy control group) | | | | | | | |

It was also confirmed that the COVAS value of the subjects of chronic fatigue syndrome/myalgic encephalomyelitis was significantly larger than the COVAS value of the healthy controls even in the section of 16000 to 30000 msec. Therefore, healthy controls and chronic fatigue syndrome/myalgic encephalomyelitis group can be easily differentiated by the measurement of COVAS.

(3) Comparison with fibromyalgia group:
The results are shown in Fig. 7. While the subjects of chronic fatigue syndrome/myalgic encephalomyelitis showed a similar waveform to the subjects of fibromyalgia, they also showed high peak intensity of pain.

Next, the mean values of COVAS of the healthy controls and the subjects of chronic fatigue syndrome/myalgic encephalomyelitis in each section of 30000 to 45000 msec, 45000 to 60000 msec, and 30000 to 60000 msec were analyzed by Welch's t-test. The results are shown in the table below.

**[Table 14]**

| Comparison of FM and chronic fatigue syndrome/myalgic encephalomyelitis. | | | | | | | |
|---|---|---|---|---|---|---|---|
| | | N | M | SD | t | r | P |
| 30000-45000 | FM | 133 | 48.57 | 34.39 | 1.40 | 0.09 | 0.16 |
| | Chronic fatigue | 90 | 54.87 | 30.47 | | | |
| 45000-60000 | FM | 133 | 38.84 | 33.04 | 0.17 | 0.01 | 0.86 |
| | Chronic fatigue | 90 | 38.09 | 31.09 | | | |
| 30000-60000 | FM | 133 | 43.78 | 32.61 | 0.63 | 0.04 | 0.53 |
| | Chronic fatigue | 90 | 546.47 | 29.80 | | | |

| | | | | | | | |
|---|---|---|---|---|---|---|---|
| (FM: Fibromyalgia group, Chronic fatigue: Chronic fatigue syndrome/myalgic encephalomyelitis group) | | | | | | | |

While there was a tendency that the COVAS value of the subjects of chronic fatigue syndrome/myalgic encephalomyelitis slightly increased in the section of 30000 to 45000 msec, no significant differences were observed in the COVAS values in the sections of 45000 to 60000 msec and 30000 to 60000 msec.

Furthermore, in the section of 16000 to 30000 msec, the mean value of COVAS of the chronic fatigue syndrome/myalgic encephalomyelitis group (N=91) and the mean value of COVAS of the fibromyalgia group (N=115) were analyzed by t-test. The results are shown in the table below.

**[Table 15]**

| 16000-30000msec | | | | | | |
|---|---|---|---|---|---|---|
| | N | Mean | SD | t | r | p |
| chronic fatigue | 91 | 71.22 | 23.84 | -2.78 | .20 | .006 (p<.01) |
| FM | 115 | 59.14 | 38.06 | | | |

| | | | | | | |
|---|---|---|---|---|---|---|
| (FM: Fibromyalgia group, chronic fatigue: Chronic fatigue syndrome/myalgic encephalomyelitis group) | | | | | | |

The COVAS value of the chronic fatigue syndrome/myalgic encephalomyelitis group was significantly larger in the section of 16000 to 30000 msec. Therefore, it was shown that fibromyalgia and chronic fatigue syndrome/myalgic encephalomyelitis can be easily differentiated by comparing the peak intensity of pain.

In addition, there were no significant correlations between the values of J-FIQ, State-Trait Anxiety Inventory (STAI), Beck Depression Inventory (BDI), and the COVAS value of the chronic fatigue syndrome/myalgic encephalomyelitis group (30000 msec to 60000 msec, N=90).

### [INDUSTRIAL APPLICABILITY]

According to the present invention, a patient having pain other than neuropathic pain which has heretofore been difficult to differentiate can be differentiated in a short time by a single, convenient and noninvasive test. In particular, the system of the present invention enables to provide clear judgement criteria for diseases which are difficult to differentiate so far and which are sometimes identifiable or misdiagnosed, thus enabling to provide more effective treatments. In addition, the present invention also provides new diagnostic criteria for diseases for which no established biological marker exists; according to the system of the present invention, a possibility to accurately and conveniently diagnose pain with unknown causes, which has conventionally been difficult to diagnose, can be increased.

## Claims

1. A diagnostic system for diagnosing a disease requiring differentiation from chronic pain-related diseases in a subject, wherein the disease is selected from depression, rheumatism and chronic fatigue syndrome/myalgic encephalomyelitis, wherein the diagnostic system comprises
a temperature stimulus generation part,
an input part for inputting information relating to a temperature stimulus generated in the temperature stimulus generation part, and
an analysis part for analyzing the inputted information,
wherein said temperature stimulus generation part is configured to generate a warm sensation stimulus based on a temperature change control program, and the analysis part is an arithmetic processing device configured to analyze the information inputted from the input part, to compare it with a reference information and to diagnose the disease requiring differentiation from chronic pain-related diseases based on a comparison of the inputted information and the reference information, wherein the temperature stimulus generation part is configured to apply various temperature stimuli to a subject's skin and to change its temperature, wherein the temperature stimulus generated at the temperature stimulus generation part is generated by a temperature change of this part, and such temperature change is managed by the temperature change control program for a pain offset test comprising
(1) a step of changing the temperature of the stimulus generation part from room temperature to a first temperature higher than room temperature,
(2) a step of keeping the temperature of the stimulus generation part at the first temperature for a while,
(3) a step of changing the temperature of the stimulus generation part to a second temperature higher than the first temperature, and then keeping it for a while,
(4) a step of changing the temperature of the stimulus generation part to the first temperature, and then keeping it for a while,
(5) a step of returning the temperature of the stimulus generation part to room temperature,
wherein the first temperature is in the range of 40 to 45°C and the difference between the first temperature and the second temperature is in the range of 1 to 5°C,
wherein the information relating to temperature stimulus includes the degree of warm sensation stimulus felt by the subject and the degree of pain stimulus when the temperature stimulus is recognized as a pain stimulus,
wherein the analysis part is configured to diagnose that the subject has chronic fatigue syndrome/myalgic encephalomyelitis when a degree of a peak of pain and a degree of the pain after offset analyzed are both statistically significantly higher than those of healthy controls as reference values or
wherein the analysis part is configured to diagnose that the subject has depression when the degree of the peak of pain analyzed is statistically significantly lower than a reference value or
wherein the analysis part is configured to diagnose that the subject has rheumatism when the degree of the peak of pain analyzed is statistically significantly lower than that of healthy controls as reference value, and the degree of the pain after offset is statistically significantly lower than that of healthy controls as reference value.

2. The diagnostic system according to claim 1, wherein the temperature stimulus generation part has a sensor for measuring temperature of a surface of the skin.

3. The diagnostic system according to claim 1 or 2 wherein the first temperature is in the range of 43 to 45°C.

4. The diagnostic system according to any of the preceding claims, wherein the difference between the first temperature and the second temperature is in the range of 1 to 3°C.

## Patentansprüche

1. Diagnosesystem zum Diagnostizieren einer Krankheit, die eine Differenzierung von chronischen schmerzassoziierten Krankheiten bei einer Person erfordert, wobei die Krankheit ausgewählt ist aus Depression, Rheuma und chronischem Erschöpfungssyndrom/myalgischer Enzephalomyelitis, wobei das Diagnosesystem umfasst
ein Temperaturreiz-Erzeugungsteil,
ein Eingabeteil zum Eingeben von Informationen, die sich auf einen Temperaturreiz beziehen, der in dem Temperaturreiz-Erzeugungsteil erzeugt wird, und
ein Analyseteil zum Analysieren der eingegebenen Information,
wobei das Temperaturreiz-Erzeugungsteil so konfiguriert ist, dass ein Wärmeempfindungsreiz auf der Grundlage eines Temperaturänderungs-Steuerprogramms erzeugt wird und der Analyseteil eine arithmetische Verarbeitungsvorrichtung ist, die so konfiguriert ist, dass sie die eingegebene Information von dem Eingabeteil analysiert, sie mit einer Referenzinformation vergleicht und die Krankheit, die eine Differenzierung von chronischen schmerzassoziierten Krankheiten erfordert, auf der Grundlage eines Vergleichs der eingegebenen Information und der Referenzinformation diagnostiziert, wobei das Temperaturreiz-Erzeugungsteil so konfiguriert ist, dass es verschiedene Temperaturreize auf die Haut einer Person aufbringt und seine Temperatur ändert, wobei der an dem Temperaturreiz-Erzeugungsteil erzeugte Temperaturreiz durch eine Temperaturänderung dieses Teils erzeugt wird und eine solche Temperaturänderung durch das Temperaturänderungs-Steuerprogramm für einen Schmerz-Offset-Test gesteuert wird, der Folgendes umfasst
(1) einen Schritt des Änderns der Temperatur des Reiz-Erzeugungsteils von Raumtemperatur auf eine erste Temperatur, die höher als die Raumtemperatur ist,
(2) einen Schritt des Haltens der Temperatur des Reiz-Erzeugungsteils auf der ersten Temperatur für eine Weile,
(3) einen Schritt des Änderns der Temperatur des Reiz-Erzeugungsteils auf eine zweite Temperatur, die höher als die erste Temperatur ist, und des anschließenden Haltens derselben für eine Weile,
(4) einen Schritt des Änderns der Temperatur des Reiz-Erzeugungsteils auf die erste Temperatur und des anschließenden Haltens derselben für eine Weile,
(5) einen Schritt des Zurückbringens der Temperatur des Reiz-Erzeugungsteils auf Raumtemperatur,
wobei die erste Temperatur im Bereich von 40 bis 45°C liegt und der Unterschied zwischen der ersten Temperatur und der zweiten Temperatur im Bereich von 1 bis 5°C liegt,
wobei die Information, die sich auf den Temperaturreiz bezieht, den Grad des von der Person empfundenen Wärmeempfindungsreizes und den Grad des Schmerzreizes, wenn der Temperaturreiz als ein Schmerzreiz erkannt wird, umfasst
wobei der Analyseteil so konfiguriert ist, dass er diagnostiziert, dass die Person ein chronisches Erschöpfungssyndrom/myalgische Enzephalomyelitis hat, wenn ein Grad einer Schmerzspitze und ein Grad des Schmerzes nach dem analysierten Offset beide statistisch signifikant höher sind als diejenigen von gesunden Kontrollen als Referenzwerte oder
wobei der Analyseteil so konfiguriert ist, dass er diagnostiziert, dass die Person eine Depression hat, wenn der Grad der analysierten Schmerzspitze statistisch signifikant niedriger als ein Referenzwert ist, oder
wobei der Analyseteil so konfiguriert ist, dass er diagnostiziert, dass die Person Rheuma hat, wenn der Grad der analysierten Schmerzspitze statistisch signifikant niedriger ist als derjenige von gesunden Kontrollen als Referenzwert, und der Grad des Schmerzes nach dem Offset statistisch signifikant niedriger ist als derjenige von gesunden Kontrollen als Referenzwert.

2. Diagnosesystem nach Anspruch 1, wobei der Temperaturreiz-Erzeugungsteil einen Sensor zur Messung der Temperatur einer Hautoberfläche aufweist.

3. Diagnosesystem nach Anspruch 1 oder 2, wobei die erste Temperatur im Bereich von 43 bis 45°C liegt.

4. Diagnosesystem nach einem der vorhergehenden Ansprüche, wobei die Differenz zwischen der ersten Temperatur und der zweiten Temperatur im Bereich von 1 bis 3°C liegt.

## Revendications

1. Système de diagnostic pour diagnostiquer une maladie nécessitant une différenciation de maladies liées à la douleur chronique chez un sujet, dans lequel la maladie est sélectionnée parmi la dépression, le rhumatisme et le syndrome de fatigue chronique/l'encéphalomyélite myalgique, dans lequel le système de diagnostic comprend
une partie de génération de stimulus de température,
une partie de saisie pour saisir des informations concernant un stimulus de température généré dans la partie de génération de stimulus de température, et
une partie d'analyse pour analyser les informations saisies,
dans lequel ladite partie de génération de stimulus de température est configurée pour générer un stimulus de sensation chaude basé sur un programme de commande de changement de température, et la partie d'analyse est un dispositif de traitement arithmétique configuré pour analyser les informations saisies depuis la partie de saisie, pour les comparer à des informations de référence et pour diagnostiquer la maladie nécessitant une différenciation de maladies liées à la douleur chronique en fonction d'une comparaison des informations saisies et des informations de référence, dans lequel la partie de génération de stimulus de température est configurée pour appliquer divers stimuli de température à la peau d'un sujet et pour changer sa température, dans lequel le stimulus de température généré au niveau de la partie de génération de stimulus de température est généré par un changement de température de cette partie, et un tel changement de température est géré par le programme de commande de changement de température pour un test de compensation de la douleur comprenant
(1) une étape de changement de la température de la partie de génération de stimulus de la température ambiante à une première température supérieure à la température ambiante,
(2) une étape de maintien de la température de la partie de génération de température à la première température pendant un moment,
(3) une étape de changement de la température de la partie de génération de stimulus à une seconde température supérieure à la première température, puis de son maintien pendant un moment,
(4) une étape de changement de la température de la partie de génération de stimulus à la première température, puis de son maintien pendant un moment,
(5) une étape de retour de la température de la partie de génération de stimulus à la température ambiante,
dans lequel la première température est dans la plage de 40 à 45 °C, et la différence entre la première température et la seconde température est dans la plage de 1 à 5 °C,
dans lequel les informations concernant un stimulus de température incluent le degré de stimulus de sensation chaude ressenti par le sujet et le degré de stimulus de douleur lorsque le stimulus de température est reconnu comme un stimulus de douleur,
dans lequel la partie d'analyse est configurée pour diagnostiquer que le sujet a un syndrome de fatigue chronique/une encéphalomyélite myalgique lorsqu'un degré d'un pic de douleur et un degré de la douleur après compensation analysés sont tous deux statistiquement nettement supérieurs à ceux de témoins sains comme valeurs de référence ou
dans lequel la partie d'analyse est configurée pour diagnostiquer que le sujet a une dépression lorsque le degré du pic de douleur analysé est statistiquement nettement inférieur à une valeur de référence ou
dans lequel la partie d'analyse est configurée pour diagnostiquer que le sujet a un rhumatisme lorsque le degré du pic de douleur analysé est statistiquement nettement inférieur à celui de témoins sains comme valeur de référence, et le degré de la douleur après compensation est statistiquement nettement inférieur à celui de témoins sains comme valeur de référence.

2. Système de diagnostic selon la revendication 1, dans lequel la partie de génération de stimulus de température a un capteur pour mesurer la température d'une surface de la peau.

3. Système de diagnostic selon la revendication 1 ou 2, dans lequel la première température est dans la plage de 43 à 45 °C.

4. Système de diagnostic selon l'une quelconque des revendications précédentes, dans lequel la différence entre la première température et la seconde température est dans la plage de 1 à 3 °C.
